Europäisches Patentamt

European Patent Office

Office européen des brevets

(11) Veröffentlichungsnummer: **0 664 280 A1**

## (12) EUROPÄISCHE PATENTANMELDUNG

(21) Anmeldenummer: **94118952.4**

(22) Anmeldetag: **01.12.94**

(51) Int. Cl.[6]: **C07C 57/04**, C07C 51/42, B01D 61/36

(30) Priorität: **19.01.94 DE 4401405**

(43) Veröffentlichungstag der Anmeldung: **26.07.95 Patentblatt 95/30**

(84) Benannte Vertragsstaaten: **BE DE FR GB IT**

(71) Anmelder: **CHEMISCHE WERKE HÜLS AG - RSP Patente / PB 15 - D-45764 Marl (DE)**

(72) Erfinder: **Sridhar, Srinivasan, Dr. Lehmbecker Pfad 52 D-45770 Marl (DE)**

(54) **Verfahren zur Entwässerung von Acrylsäure.**

(57) Die Entwässerung von wäßrigen Acrylsäurelösungen wird dadurch vorgenommen, daß man in einer Pervaporation 10 bis 90 % des Wassers entfernt. Dadurch wird eine nachfolgende destillative Aufarbeitung der Acrylsäure entlastet.

Abb. 1

EP 0 664 280 A1

Die vorliegende Erfindung betrifft ein neues Verfahren zur Entwässerung von wäßrigen Acrylsäurelösungen.

Acrylsäure kann auf verschiedene Weisen großtechnisch hergestellt werden, wobei die Verfahren von dem Ausgangsstoff abhängen. Nach US 2 922 815 kann Acrylsäure aus Acetylen, Kohlenmonoxid und Wasser hergestellt werden. Dabei bleibt das Nasser als Überschuß im Produktstrom. Auch bei der Hydrolyse von Acrylnitril zu Acrylsäure gemäß US 2 734 915 wird Wasser verwendet. Folglich fällt auch hier neben dem Produkt überschüssiges Wasser an. Eine weitere Möglichkeit ist nach US 4 537 874 die Luftoxidation von Propylen zu Acrolein und anschließende weitere Oxidation zu Acrylsäure. Durch Zufuhr von Wasser wird hier die Reaktionstemperatur drastisch gesenkt (Quenchen), um die Reaktion zu beenden. Bei diesem Verfahren fällt ebenfalls Wasser als Nebenprodukt an. Bei den verschiedenen Verfahren muß das Produkt anschließend nicht nur von den nicht abreagierten gasförmigen Komponenten, sondern auch vom zugeführten bzw. entstandenen Wasser abgetrennt werden.

Es sind verschiedene Verfahrensvarianten vorgeschlagen worden, die die Entwässerung der Acrylsäure verwirklichen. Sofern der Wasseranteil recht hoch ist, kann sich eine direkte Destillation des Wassers als unwirtschaftlich erweisen. Es sind verschiedene Verfahren bekannt, die Entwässerung durch einen Extraktionsschritt zu bewirken, wobei als Extraktionsmittel beispielsweise Ethylacetat (GB 995 472), Diisopropylether (GB 1 081 095), Alkylacrylate, Ketone, $\beta$-Alkyloxypropionat, Derivate von Cyclohexanon (US 3 657 332) oder Pyrrolidon verwendet werden. Acrylsäure wird durch das Extraktionsmittel aufgenommen und daraus destillativ oder mittels Extraktivdestillation, Kristallisation oder Azeotropdestillation wiedergewonnen. Man kann bei einem Wassergehalt von < 60 % im Ausgangsgemisch auch den Extraktionsschritt umgehen und die Lösung direkt durch Azeotropdestillation entwässern. Dabei ist jedoch wegen der thermischen Belastung auch dann, wenn Acrylsäure stabilisiert wird, die Gefahr einer Verlegung (Verstopfung) durch Polymerisation gegeben. Eine Destillation bei niedriger Temperatur unter Normaldruck würde außerdem zu hohem Dampfvolumen und folglich zu großen Kolonnen führen. Sinnvoll wäre eine vorgeschaltete Verfahrensstufe, die den Wasseranteil in der Lösung bei milden Bedingungen deutlich, beispielsweise auf < 40 %, erniedrigt, damit die destillative Entwässerung sich nur auf einen geringen Wasseranteil beschränkt.

Pervaporation ist eine Methode, bei der eine semipermeable Membran als eine Barriere für den organischen Stoff bei dem Übergang von der flüssigen in die Dampfphase eingeführt wird. Da die Membran bevorzugt Wasser durchläßt, wird die Trennwirkung deutlich begünstigt. Besonders in den letzten Jahren sind zahlreiche Anwendungen der Pervaporation, beispielsweise zur Entwässerung von organischen Lösemitteln, Alkoholen und Aminen, bekannt geworden, wobei eine Membran, im wesentlichen bestehend aus einer Trennschicht aus Polyvinylalkohol (PVA), die selektive Abtrennung des Wassers als Dampfphase ermöglicht.

Nach Q. T. Nguyen, Proc. Int. Conf. Pervaporation Processes Chem. Ind. 5th, 67 - 78, (1991), sind jedoch übliche PVA-Membranen für die Pervaporation einer wäßrigen Essigsäure bei > 80 °C ungeeignet, da sich die Membran dabei auflöst. Das gleiche gilt im besonderen auch für die noch aggressivere wäßrige Mischung aus Essigsäure und Chloressigsäure. Diese starken organischen Säuren können nur mit Hilfe von Spezialmembranen aufkonzentriert werden. Dementsprechend wird in US 4 971 699 für die Pervaporation von wäßriger Ameisen- oder Essigsäure eine spezielle Membran aus PVA, Polyacrylat und Polysulfon verwendet. Nach M. Yoshikawa, J. Membrane Sci. 82 (1993), 157 - 62, kann eine wäßrige Essigsäure auch mit Hilfe einer Membran aus Acrylsäure-Acrylnitril-Copolymeren pervaporisiert werden.

Demnach wird die Pervaporation einer wäßrigen Essigsäure oder einer wäßrigen, noch stärkeren Säure mit Hilfe von PVA-Membranen nicht empfohlen.

Aufgabe der vorliegenden Erfindung war es nun, wäßrige Acrylsäurelösungen ohne Extraktionsmittel und bei geringem Energieaufwand schonend aufzukonzentrieren.

Die Aufgabe wird überraschend dadurch gelöst, daß man 10 bis 90 % des Wassers durch Pervaporation mit Hilfe einer Membran entfernt.

Das erfindungsgemäße Verfahren kann mit Hilfe einfacher PVA-Membran ausgeführt werden. Das überrascht deshalb, weil Acrylsäure mit $pK_a$ = 4,26 eine noch stärkere Säure als Essigsäure mit $pK_a$ = 4,76 ist und deshalb noch aggressiver sein sollte. Daher war zu befürchten, daß eine Entwässerung von Acrylsäure mit einer herkömmlichen PVA-Membran bei den erforderlichen Temperaturen nicht möglich ist. Gemäß o. g. Erfahrung wäre die Entwicklung einer Membran aus anderen resistenten Materialien notwendig.

Die Pervaporation wird vorzugsweise bei 60 bis 100 °C durchgeführt, wobei Temperaturen von 70 bis 95 °C besonders bevorzugt eingestellt werden.

Der retentatseitige Druck beträgt vorzugsweise 0,1 bis 0,2 MPa, wobei meist bei Normaldruck gearbeitet wird. Der permeatseitige Druck auf der anderen Seite der Membran liegt vorzugsweise bei 1 bis 900, im besonderen bei 70 bis 200 hPa. Es wird also vorzugsweise ein leichtes Vakuum eingestellt. In einer speziellen Ausführungsform wird permeatseitig ein Inertgas, wie z. B. Stickstoff, bei Normaldruck oder Vakuum verwendet.

Geeignete Membranen bestehen beispielsweise aus PVA, Polyimid, Polysulfon, Polyphenylenoxid, PVC, Vinylalkohol/Vinylacetat-, Acrylsäure/Acrylnitril-, Vinylpyridin/Acrylnitril- oder Vinylacetat/Vinylpyrrolidon-Copolymeren. Wegen einer hohen Permeabilität und Selektivität bei ausreichender Stabilität verwendet man vorzugsweise Membranen mit einer aus PVA oder einer zu über 90 % aus PVA bestehenden Trennschicht.

Für die Pervaporation werden vorzugsweise 20- bis 70%ige wäßrige Acrylsäurelösungen eingesetzt, wobei die Konzentration im besonderen 40 bis 60 % beträgt. Die aufkonzentrierte Lösung, das Retentat, ist vorzugsweise 60- bis 95%ig. Besonders bevorzugt stellt man bei der Pervaporation 70- bis 80%ige Acrylsäurelösungen her. Das Permeat ist im Idealfall frei von Acrylsäure. In der Praxis liegt der Acrylsäuregehalt im Permeat vorzugsweise bei < 20 %, wobei die Konzentrationen besonders bevorzugt bei 1 bis 10 % liegen.

Die Pervaporation kann kontinuierlich oder diskontinuierlich durchgeführt werden. Man kann dabei eine ein- oder eine mehrstufige Pervaporation , beispielsweise eine Kaskade von Pervaporationsschritten, vornehmen. Bei Entwässerungen in mehreren Verfahrensstufen kann die Acrylsäure im Permeat durch Pervaporation oder auch durch Umkehrosmose aufkonzentriert werden. In einer besonderen Ausführungsform wird bei einer mehrstufigen Entwässerung mindestens in der 1. Stufe eine Pervaporation und mindestens in der letzten Stufe, gegebenenfalls auch in den Stufen davor, jedoch nicht in der 1. Stufe eine Umkehrosmose durchgeführt. Gemäß dieser speziellen Verfahrensweise wird also beispielsweise bei einer Entwässerung in 4 Verfahrensstufen in der 4. und gegebenenfalls auch in der 2. und 3. Stufe eine Umkehrosmose vorgenommen.

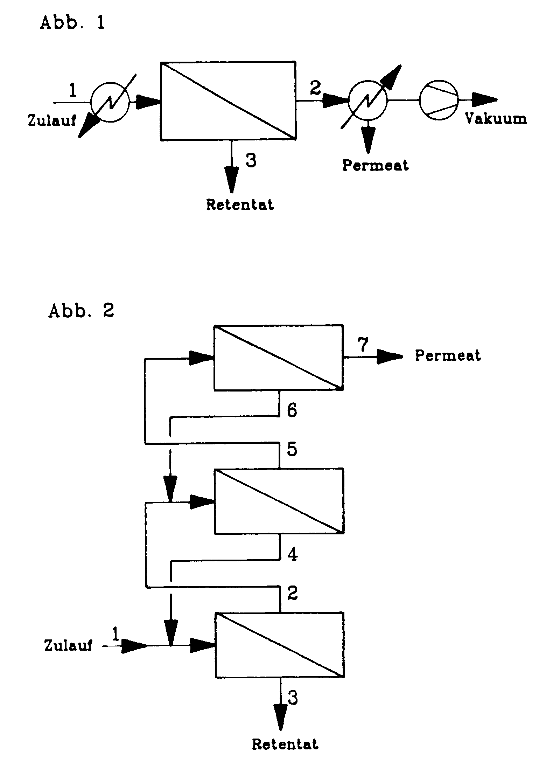

Eine einstufige Pervaporation ist in Abbildung 1 dargestellt. Bedingt durch das transmembrane Gefälle in der Wasserkonzentration tritt hier das Wasser aus der Lösung durch die Membran in die Permeatzone, während Acrylsäure von der Membran zurückgehalten wird. Das wasserreiche Permeat wird als Strom 2 abgezogen, während die Säure überwiegend im Retentatstrom 3 anfällt. Wenn ein bedeutender Anteil an Acrylsäure durch die Membran dringt, kann die Pervaporation auch mehrstufig als Kaskade ausgeführt werden, damit das Permeat aus der letzten Stufe säurefrei (< 1 %) ist. Gleichzeitig kann dadurch das Retentat der ersten Stufe an Säure angereichert werden. In der Abbildung 2 wird eine 3stufige Kaskade dargestellt, bei der beispielsweise aus einem Ausgangsgemisch (Strom 1) mit 50 % Wasser ein Retentat (Strom 3) mit 30 % Wasser und ein Endpermeat (Strom 7) mit < 1 % Säure gewonnen werden kann. Das Permeat aus der 1. Stufe (Strom 2) wird einer 2. Stufe zugeführt und das Retentat aus der 2. Stufe (Strom 4) der 1. Stufe zurückgeführt. Ähnlich wird auch zwischen der 2. und der 3. Stufe vorgegangen. Aus der 3. Stufe erscheint das Endpermeat. Nach jeder Stufe kann das Permeat entweder kondensiert werden oder in dampfförmigem Zustand erhalten bleiben. Im ersten Fall wird das Kondensat vor der Zufuhr in die nächste Stufe wieder verdampft, im zweiten Fall wird der Dampf auf Normaldruck komprimiert und in die nächste Stufe dampfförmig eingeführt ("Dampfpermeation").

Aus wirtschaftlichen Gründen ist es oft sinnvoll, die Pervaporation nur soweit zu betreiben, daß das Endpermeat einen Säuregehalt von 1 bis 5 % aufweist, wodurch eine weitere Verdampfung zum Zwecke der Abtrennung der letzten Säurereste vermieden wird. Anstelle einer weiteren Pervaporation wird das Endpermeat dann einer Umkehrosmose unterworfen, die bei Normaltemperatur und ohne Verdampfung des Permeats erfolgt. Dadurch kann ein Permeat mit einem Säuregehalt < 1 % gewonnen werden, während das Retentat des Umkehrosmoseschritts, das an Säure angereichert ist, dem Pervaporationsschritt zurückgeführt wird.

Das erfindungsgemäße Verfahren liefert bei geringem energetischen Aufwand unter schonenden Bedingungen aufkonzentrierte Acrylsäurelösungen. Bei Anwendung der Pervaporation als Zwischenstufe wird die abschließende Destillation zur vollständigen Wasserabtrennung deutlich entlastet.

Die folgenden Beispiele sollen die Erfindung verdeutlichen. Dabei wird in den Beispielen 1 bis 7 eine einstufige Pervaporation durchgeführt. In den Beispielen 8 und 9 wird von einer 3stufigen Kaskade nur die 3. Stufe betrachtet.

Angewandte Analysemethoden

Wassergehalt: Karl-Fischer-Titration
Säuregehalt: Säurezahl-Bestimmung und GC

Beispiel 1

20 cm$^2$ einer PVA-Membran (Typ 1005 der Firma GFT, Deutsche Carbone GmbH, D-66540 Neunkirchen) werden in eine Pervaporationszelle eingelegt. Vor dem ersten Einsatz wird die Membran 40 Stunden ins Wasser gelegt. Dann wird die Membran 3 Stunden lang mit einem Ausgangsgemisch, das die Acrylsäure, Wasser und Spuren von Essigsäure enthält und mit Hydrochinon stabilisiert ist, überströmt. Das Gemisch wird dabei im Kreise geführt. Der permeatseitige Druck beträgt 5 hPa, der retentatseitige Druck 0,1 MPa (Normaldruck). Um den Versuch bei konstanter Zulaufzusammensetzung durchzuführen, wird das Permeat kontinuierlich abgezogen, in einer Kühlfalle kondensiert und laufend dem Ausgangsgemisch zurückgeführt. Nach ca. 3 Stunden wird das Permeat als Probe abgeführt. In der Tabelle 1, Proben 1 bis 8, werden die Zusammensetzungen des vorgelegten Ausgangsgemisches und der Permeatprobe angegeben. Aus den Werten geht hervor, daß das Permeat grundsätzlich einen wesentlich größeren Anteil an Wasser aufweist als das Ausgangsgemisch. Bei einem hohen Wasseranteil im Bereich von > 90 % Wasser im Ausgangsgemisch werden Permeate mit < 1 % Säuren gewonnen.

Beispiel 2

Die Pervaporation wird gemäß Beispiel 1, aber bei einer tieferen Temperatur von ca. 65 °C durchgeführt. Der retentatseitige Druck (Betriebsdruck) beträgt 0,12 MPa.
Die Ergebnisse gehen aus der Tabelle 1, Proben 9 bis 12, hervor.

Beispiel 3

Die Pervaporation wird gemäß Beispiel 1, aber mit einer anderen PVA-Membran (Typ 1006 der Firma GFT, Deutsche Carbone GmbH) durchgeführt. Der permeatseitige Druck beträgt 5 hPa, der retentatseitige Druck 0,12 MPa. Die Werte sind in Tabelle 1, Proben 13 bis 17, zusammengestellt.

Beispiel 4

Die Pervaporation wird gemäß Beispiel 1, aber mit der Membran von Beispiel 3 und einem erhöhten permeatseitigen Druck von 200 hPa und bei 70 bzw. 80 °C durchgeführt.
Die Werte sind in Tabelle 1, Proben 18 und 19, aufgelistet.

Beispiel 5

Die Pervaporation wird mit einem Ausgangsgemisch bestehend aus 91,7 Gewichtsprozent Wasser und 8,3 Gewichtsprozent Acrylsäure gemäß Beispiel 1, jedoch bei einer erhöhten Temperatur von 90 °C durchgeführt. Der Säuregehalt im Permeat beträgt 1 Gewichtsprozent. Die Permeatleistung entspricht 3,55 kg/h•m$^2$.

Beispiel 6

Die Pervaporation wird gemäß Beispiel 1, aber bei einem erhöhten permeatseitigen Druck von 100 hPa durchgeführt. Das Ausgangsgemisch besteht aus 35,5 Gewichtsprozent Wasser, 0,3 Gewichtsprozent Essigsäure und 64,2 Gewichtsprozent Acrylsäure. Das Permeat enthält 69,3 Gewichtsprozent Wasser, 0,04 Gewichtsprozent Essigsäure und 30,7 Gewichtsprozent Acrylsäure. Die Permeatleistung entspricht 2,3 kg/h•m$^2$.

Beispiel 7

Die Pervaporation wird gemäß Beispiel 1, aber bei einem erhöhten permeatseitigen Druck von 200 hPa durchgeführt. Das Ausgangsgemisch besteht aus 35,8 Gewichtsprozent Wasser, 0,3 Gewichtsprozent Essigsäure und 63,9 Gewichtsprozent Acrylsäure. Der Wassergehalt im Permeat beträgt 68,0 Gewichtsprozent. Die Permeatleistung entspricht 1,5 kg/h•m$^2$.

Beispiel 8

Ein Ausgangsgemisch mit 1 Gewichtsprozent Acrylsäure und 99 Gewichtsprozent Wasser wird bei 5 MPa und 35 °C einer Umkehrosmose unterworfen. Dabei werden 36 cm² von 3 verschiedenen Membranen (Typ DESAL-DSG, -SE und - PS der Firma DESALINATION, Escondido, Kalifornien, USA) eingesetzt. Die Permeatwerte für Wasser bei 35 °C und 5 MPa betragen dabei für
DESAL-DSG 121,7 kg/h•m²
DESAL-SE 63,3 kg/h•m²
DESAL-PS 65,3 kg/h•m²
Die vorgelegte Säure wird auf diese Weise auf 2,4 Gewichtsprozent Säuregehalt (im Retentat) aufkonzentriert. Der Säuregehalt im Permeat beträgt meist 0,2 bis 0,4 Gewichtsprozent, und die Flußrate liegt im Bereich von 16 bis 40 kg/h•m². Die Ergebnisse werden in Tabelle 2 zusammengestellt.

Beispiel 9

Gemäß Beispiel 8 wird eine Umkehrosmose durchgeführt, wobei die Aufkonzentrierung in 38 Stunden bis zu 6 Gewichtsprozent Säure erfolgt. Der Säuregehalt im Permeat beträgt im Schnitt 0,2 bis 0,9 Gewichtsprozent, und die Flußrate entspricht 7 bis 40 kg/h•m². Die Ergebnisse sind der Tabelle 3 zu entnehmen.

Die in den Beispielen und den folgenden Tabellen angegebenen Temperaturen zur Pervaporation beziehen sich auf den Zulauf. An der Membran herrscht jeweils eine um ca. 5 °C niedrigere Temperatur.

Tabelle 1

| Probe | Ausgangsgemisch [Gew.-%] | | | Permeat [Gew.-%] | | | Permeat-rate in [kg/h·m²] | Tempe-ratur [°C] | Versuchs-dauer [h] |
|---|---|---|---|---|---|---|---|---|---|
| | Wasser | Essig-säure | Acryl-säure | Wasser | Essig-säure | Acryl-säure | | | |
| 1 | 11,2 | 0,3 | 88,6 | 63,9 | 0,2 | 35,9 | 1,05 | 70 | 2,5 |
| 2 | 28,9 | 0,3 | 70,8 | 74,4 | 0,1 | 25,5 | 2,15 | 72 | 3 |
| 3 | 37,8 | 0,8 | 61,4 | 80,3 | 0,3 | 19,4 | 2,04 | 73 | 2,67 |
| 4 | 51,4 | 1,5 | 47,1 | 88,0 | - | 11,6 | 2,45 | 70 | 2,2 |
| 5 | 59,3 | 1,3 | 39,4 | 91,6 | 0,3 | 8,1 | 2,48 | 70 | 2,5 |
| 6 | 68,3 | 1,1 | 30,6 | 93,8 | 0,2 | 6,0 | 3,00 | 74 | 2,5 |
| 7 | 83,6 | 0,5 | 15,9 | 97,6 | - | 2,4 | 2,11 | 74 | 2,5 |
| 8 | 96,4 | 0,1 | 3,8 | 100,0 | - | 0,15 | 1,49 | 74 | 2,5 |
| 9 | 10,3 | 0,4 | 89,4 | 60,2 | 0,2 | 39,6 | 0,85 | 65 | |
| 10 | 29,3 | 0,8 | 68,8 | 77,7 | 0,3 | 22,0 | 1,49 | 65 | |
| 11 | 41,1 | 1,2 | 57,7 | 83,2 | 0,4 | 16,5 | 1,95 | 67 | |

EP 0 664 280 A1

| Probe | Ausgangsgemisch [Gew.-%] | | | Permeat [Gew.-%] | | | Permeat- rate in [kg/h·m²] | Tempe- ratur [°C] | Versuchs- dauer [h] |
|---|---|---|---|---|---|---|---|---|---|
| | Wasser | Essig- säure | Acryl- säure | Wasser | Essig- säure | Acryl- säure | | | |
| 12 | 58,1 | 1,5 | 40,3 | 91,3 | 0,3 | 8,4 | 2,20 | 67 | |
| 13 | 30,6 | 1,6 | 67,8 | 48,2 | 0,9 | 50,3 | | 73 | |
| 14 | 40,7 | 1,4 | 57,9 | 59,6 | 1,0 | 37,4 | | 74 | |
| 15 | 46,5 | 1,8 | 51,6 | 66,3 | 1,2 | 32,8 | | 70 | |
| 16 | 49,5 | 3,2 | 47,2 | 66,8 | 1,7 | 31,4 | | 75 | |
| 17 | 69,7 | 1,1 | 29,2 | 79,8 | 0,7 | 19,5 | | 75 | |
| 18 | 90,9 | - | 9,1 | 87,3 | - | 6,6 | 4,19 | 80 | |
| 19 | 93,4 | - | 6,1 | 94,9 | - | 5,1 | 2,38 | 70 | |

## Tabelle 2

| Probe | Zeit [h] | Retentat Acrylsäure [%] | Permeat | | | | | |
|---|---|---|---|---|---|---|---|---|
| | | | DESAL-DSG | | DESAL-SE | | DESAL-PS | |
| | | | Acrylsäure [%] | Permeat-rate [kg/h·m²] | Acrylsäure [%] | Permeat-rate [kg/h·m²] | Acrylsäure [%] | Permeat-rate [kg/h·m²] |
| 1 | 0 | 1,0 | 0,32 | 39,7 | 0,2 | 22,9 | 0,17 | 25,7 |
| 2 | 2 | 1,2 | 0,38 | 36,5 | 0,25 | 21,1 | 0,25 | 28,3 |
| 3 | 5,5 | 2,4 | 0,54 | 27,7 | 0,36 | 16,4 | 0,32* | 17,9* |

\* nach 4 Stunden

EP 0 664 280 A1

Tabelle 3

| Probe | Zeit [h] | Retentat Acrylsäure [%] | Permeat DESAL-DSG Acrylsäure [%] | Permeat DESAL-DSG Permeatrate [kg/h·m²] | Permeat DESAL-SE Acrylsäure [%] | Permeat DESAL-SE Permeatrate [kg/h·m²] | Permeat DESAL-PS Acrylsäure [%] | Permeat DESAL-PS Permeatrate [kg/h·m²] |
|---|---|---|---|---|---|---|---|---|
| 1 | 0 | 1,05 | 0,28 | 39,9 | 0,18 | 26,0 | 0,15 | 30,3 |
| 2 | 2,17 | 1,22 | 0,35 | 35,9 | 0,21 | 22,8 | 0,21 | 26,9 |
| 3 | 8,50 | 1,70 | 0,45 | 31,6 | 0,26 | 19,9 | 0,28 | 23,8 |
| 4 | 15,42 | 1,96 | 0,51 | 28,6 | 0,30 | 17,5 | 0,28 | 21,6 |
| 5 | 22,33 | 2,31 | 0,55 | 27,6 | 0,32 | 16,8 | 0,31 | 20,8 |
| 6 | 29,25 | 3,04 | 0,73 | 22,8 | 0,41 | 13,3 | 0,41 | 16,6 |
| 7 | 36,25 | 4,56 | 1,27 | 16,5 | 0,72 | 8,7 | 0,68 | 11,1 |
| 8 | 38,75 | 6,02 | 1,62 | 14,6 | 0,93 | 7,1 | 0,82 | 9,4 |

**Patentansprüche**

1.  Verfahren zur Entwässerung von wäßrigen Acrylsäurelösungen, dadurch gekennzeichnet,

daß man 10 bis 90 % des Wassers durch Pervaporation mit Hilfe einer Membran entfernt.

2. Verfahren nach Anspruch 1,
dadurch gekennzeichnet,
daß man die Pervaporation bei 60 bis 100 °C, vorzugsweise bei 70 bis 95 °C, durchführt.

3. Verfahren nach Anspruch 1,
dadurch gekennzeichnet,
daß man bei der Pervaporation retentatseitig einen Druck von 0,1 bis 0,2 MPa, vorzugsweise Normaldruck, und permeatseitig einen Druck von 1 bis 900 hPa, vorzugsweise von 70 bis 200 hPa, einstellt.

4. Verfahren nach Anspruch 1,
dadurch gekennzeichnet,
daß permeatseitig ein Inertgas zugeführt wird.

5. Verfahren nach Anspruch 1,
dadurch gekennzeichnet,
daß man eine Membran mit einer aus Polyvinylalkohol oder einer zu über 90 % aus Polyvinylalkohol bestehenden Trennschicht verwendet.

6. Verfahren nach Anspruch 1,
dadurch gekennzeichnet,
daß eine 20- bis 70%ige, vorzugsweise eine 40- bis 60%ige, Lösung eingesetzt, ein 60- bis 95%iges, vorzugsweise ein 70- bis 80%iges, Retentat und ein Permeat mit < 20 %, vorzugsweise mit 1 bis 10 %, Acrylsäure erhalten wird.

7. Verfahren nach Anspruch 1,
dadurch gekennzeichnet,
daß man die Pervaporation ein- oder mehrstufig durchführt.

8. Verfahren nach Anspruch 1,
dadurch gekennzeichnet,
daß man eine mehrstufige Entwässerung durchführt und dabei mindestens in der 1. Stufe eine Pervaporation vornimmt.

9. Verfahren nach den Ansprüchen 1 und 8,
dadurch gekennzeichnet,
daß man bei einer mehrstufigen Entwässerung in der letzten Stufe und gegebenenfalls auch in den Stufen davor, jedoch nicht in der 1. Stufe, eine Umkehrosmose vornimmt.

EP 0 664 280 A1

Abb. 1

Abb. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 096 339 (GFT INGENIEURBÜRO FÜR INDUSTRIEANLAGENBAU) <br> * Seite 3, Zeile 7 – Zeile 12; Anspruch 1 * <br> --- | 1 | C07C57/04 <br> C07C51/42 <br> B01D61/36 |
| Y | US-A-4 978 430 (NAKAGAWA ET AL.) <br> * Spalte 3, Zeile 35 – Zeile 50 * <br> ----- | 1 | |

**RECHERCHIERTE SACHGEBIETE (Int.Cl.6)**

C07C

Der vorliegende Recherchenbericht wurde für alle Patentansprüche erstellt

| Recherchenort | Abschlußdatum der Recherche | Prüfer |
|---|---|---|
| DEN HAAG | 25.April 1995 | Klag, M |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
......................................................................
& : Mitglied der gleichen Patentfamilie, übereinstimmendes
    Dokument